# EUROPEAN PATENT SPECIFICATION

(11) **EP 1 395 263 B1**
(45) Date of publication and mention of the grant of the patent: **30.12.2009**
(21) Application number: 02729202.8
(22) Date of filing: 13.05.2002
(51) Int. Cl.: A61K 31/558, A61K 31/559, A61P 27/06

(54) **3, 7 OR 3 AND 7 THIA OR OXA PROSTANOIC ACID DERIVATIVES AS AGENTS FOR LOWERING INTRAOCULAR PRESSURE**
3, 7 ODER 3 UND 7 THIA- ODER OXAPROSTANSÄURE DERIVATE ALS MITTEL ZUR SENKUNG DES AUGENINNENDRUCKS
DERIVES D'ACIDE 3, 7 OU 3 ET 7 THIA OU OXA PROSTANOIQUE COMME AGENTS POUR REDUIRE LA PRESSION INTRAOCULAIRE

(30) Priority: 14.06.2001 US 882720; 20.03.2002 US 103301
(43) Date of publication of application: 10.03.2004
(73) Proprietor: ALLERGAN, INC., Irvine CA 92612 (US)
(72) Inventor: BURK, Robert, M., Laguna Beach, CA 92651 (US); HOLOBOSKI, Mark, Laguna Niguel, CA 92677 (US); POSNER, Mari, F., Laguna Niguel, CA 92677 (US)
(74) Representative: HOFFMANN EITLE
(86) International application number: PCT/US2002/015207
(87) International publication number: WO 2002/102389

(56) References cited:
- EP-A- 0 985 663
- WO-A-00/38667

## Description

### Field of the Invention

The present invention relates to 3, 7 or 3 and 7 thia or oxa prostanoic acid derivatives as potent ocular hypotensives that are particularly suited for the management of glaucoma.

### Background of the Invention

### Description of Related Art

Ocular hypotensive agents are useful in the treatment of a number of various ocular hypertensive conditions, such as post-surgical and post-laser trabeculectomy ocular hypertensive episodes, glaucoma, and as presurgical adjuncts.

Glaucoma is a disease of the eye characterized by increased intraocular pressure. On the basis of its etiology, glaucoma has been classified as primary or secondary. For example, primary glaucoma in adults (congenital glaucoma) may be either open-angle or acute or chronic angle-closure. Secondary glaucoma results from pre-existing ocular diseases such as uveitis, intraocular tumor or an enlarged cataract.

The underlying causes of primary glaucoma are not yet known. The increased intraocular tension is due to the obstruction of aqueous humor outflow. In chronic open-angle glaucoma, the anterior chamber and its anatomic structures appear normal, but drainage of the aqueous humor is impeded. In acute or chronic angle-closure glaucoma, the anterior chamber is shallow, the filtration angle is narrowed, and the iris may obstruct the trabecular meshwork at the entrance of the canal of Schlemm. Dilation of the pupil may push the root of the iris forward against the angle, and may produce pupilary block and thus precipitate an acute attack. Eyes with narrow anterior chamber angles are predisposed to acute angle-closure glaucoma attacks of various degrees of severity.

Secondary glaucoma is caused by any interference with the flow of aqueous humor from the posterior chamber into the anterior chamber and subsequently, into the canal of Schlemm. Inflammatory disease of the anterior segment may prevent aqueous escape by causing complete posterior synechia in iris bombe, and may plug the drainage channel with exudates. Other common causes are intraocular tumors, enlarged cataracts, central retinal vein occlusion, trauma to the eye, operative procedures and intraocular hemorrhage.

Considering all types together, glaucoma occurs in about 2% of all persons over the age of 40 and may be asymptotic for years before progressing to rapid loss of vision. In cases where surgery is not indicated, topical b-adrenoreceptor antagonists have traditionally been the drugs of choice for treating glaucoma.

Certain eicosanoids and their derivatives have been reported to possess ocular hypotensive activity, and have been recommended for use in glaucoma management. Eicosanoids and derivatives include numerous biologically important compounds such as prostaglandins and their derivatives. Prostaglandins can be described as derivatives of prostanoic acid which have the following structural formula:

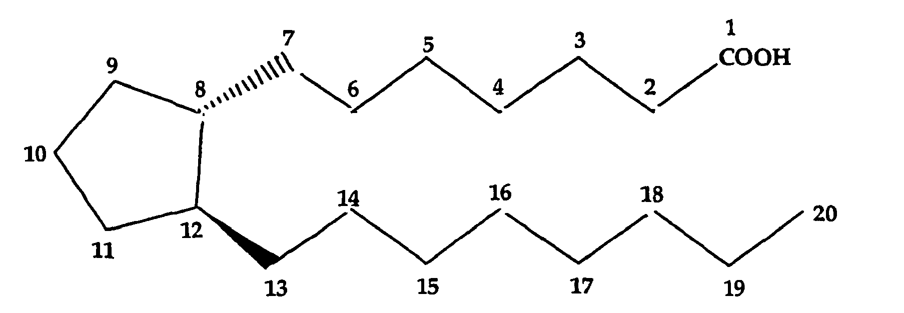

Various types of prostaglandins are known, depending on the structure and substituents carried on the alicyclic ring of the prostanoic acid skeleton. Further classification is based on the number of unsaturated bonds in the side chain indicated by numerical subscripts after the generic type of prostaglandin [e.g. prostaglandin E₁ (PGE₁), prostaglandin E₂ (PGE₂)], and on the configuration of the substituents on the alicyclic ring indicated by α or β [e.g. prostaglandin F_{2α} (PGF_{2β})].

Prostaglandins were earlier regarded as potent ocular hypertensives, however, evidence accumulated in the last decade shows that some prostaglandins are highly effective ocular hypotensive agents, and are ideally suited for the long-term medical management of glaucoma (see, for example, Bito, L.Z. Biological Protection with Prostaglandins, Cohen, M.M., ed., Boca Raton, Fla, CRC Press Inc., 1985, pp. 231-252; and Bito, L.Z., Applied Pharmacology in the Medical Treatment of Glaucomas Drance, S.M. and Neufeld, A.H. eds., New York, Grune & Stratton, 1984, pp. 477-505. Such prostaglandins include PGF_{2α}, PGF_{1α}, PGE₂, and certain lipid-soluble esters, such as C₁ to C₂ alkyl esters, e.g. 1-isopropyl ester, of such compounds.

Although the precise mechanism is not yet known experimental results indicate that the prostaglandin-induced reduction in intraocular pressure results from increased uveoscleral outflow [Nilsson et.al., Invest. Ophthalmol. Vis. Sci. (suppl), 284 (1987)].

The isopropyl ester of PGF_{2α} has been shown to have significantly greater hypotensive potency than the parent compound, presumably as a result of its more effective penetration through the cornea. In 1987, this compound was described as "the most potent ocular hypotensive agent ever reported" [see, for example, Bito, L.Z., Arch. Ophthalmol. 105, 1036 (1987), and Siebold et.al., Prodrug 5 3 (1989)].

Whereas prostaglandins appear to be devoid of significant intraocular side effects, ocular surface (conjunctival) hyperemia and foreign-body sensation have been consistently associated with the topical ocular use of such compounds, in particular PGF_{2α} and its prodrugs, e.g., its 1-isopropyl ester, in humans. The clinical potentials of prostaglandins in the management of conditions associated with increased ocular pressure, e.g. glaucoma are greatly limited by these side effects.

In a series of co-pending United States patent applications assigned to Allergan, Inc. prostaglandin esters with increased ocular hypotensive activity accompanied with no or substantially reduced side-effects are disclosed. The co-pending USSN 596,430 (filed 10 October 1990, now U.S. Patent 5,446,041), relates to certain 11-acyl-prostaglandins, such as 11-pivaloyl, 11-acetyl, 11-isobutyryl, 11-valeryl, and 11-isovaleryl PGF_{2α}. Intraocular pressure reducing 15-acyl prostaglandins are disclosed in the co-pending application USSN 175,476 (filed 29 December 1993). Similarly, 11,15- 9,15 and 9,11-diesters of prostaglandins, for example 11,15-dipivaloyl PGF_{2α} are known to have ocular hypotensive activity. See the co-pending patent applications USSN Nos. 385,645 (filed 07 July 1989, now U.S. Patent 4,994,274), 584,370 (filed 18 September 1990, now U.S. Patent 5,028,624) and 585,284 (filed 18 September 1990, now U.S. Patent 5,034,413).

WO00/38667 also discloses the use of prostaglandin E agonists for treatment of glaucoma. EP 0 985 663 discloses 3,7-dithiaprostanoic derivatives for the treatment of several diseases, not including ocular diseases.

### Summary of the Invention

The present invention concerns the use of a therapeutically effective amount of a compound of formula I 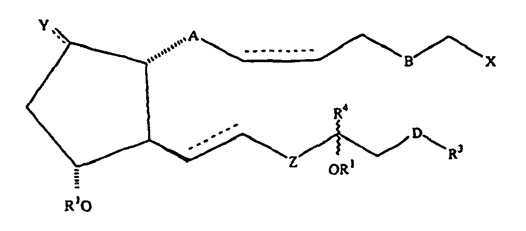 wherein hatched lines represent the α configuration, a triangle represents the β configuration, a wavy line represents either the α configuration or the β configuration and a dotted line represents the presence or absence of a double bond; A and B are independently selected from the group consisting of O, S and CH₂;
provided that at least one of A or B is S;
D represents a covalent bond or CH₂, O, S or NH;
X is CO₂R, CONR₂, CH₂OR, P(O)(OR)₂, CONRSO₂R, SONR₂ or 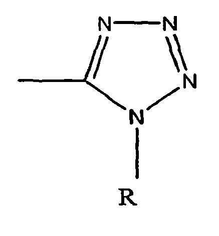 Y is O, OH, OCOR², halogen or cyano;
Z is CH₂ or a covalent bond;
R is H or R².
R¹ is H, R², phenyl, or COR²;
R² is C₁-C₅ lower alkyl or alkenyl;
R³ is benzothienyl, benzofuranyl, or substituted derivatives thereof,
wherein the substituents maybe selected from the group consisting of C₁-C₅ alkyl, halogen, CF₃ₓ CN, NO₂, NR₂, CO₂R and OR; and
R⁴ is hydrogen or C₁-C₅ alkyl, for the manufacture of a medicament for the treatment of ocular hypertension of glaucoma.

In a still further aspect, the present invention relates to a pharmaceutical product, comprising
a container adapted to dispense its contents in a metered form; and
an ophthalmic solution therein, as hereinabove defined.

Finally, certain of the compounds represented by the above formula, disclosed below and utilized in the use of the present invention are novel and unobvious.

### Brief Description of the Drawing Figures

FIG. 1 is a schematic of the chemical synthesis of a certain intermediate for the compounds of the invention as disclosed in Examples 1 through 3.
FIG. 2 is a schematic of the chemical synthesis of certain compounds related to the compounds of the invention as disclosed in Examples 4 through 7.

### Detailed Description of the Invention

The present invention relates to the use of 3, 7 and 3 and 7 thia or oxa prostanoic acid derivatives as ocular hypotensives. The compounds used in accordance with the present invention are encompassed by the following structural formula I:

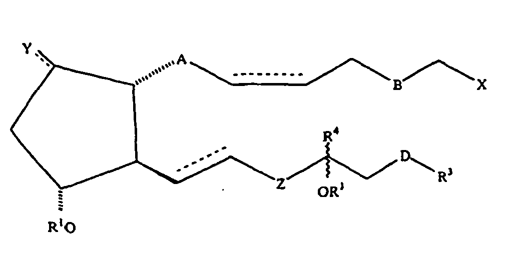

A preferred group of the compounds of the present invention includes compounds that have the following structural formula II:

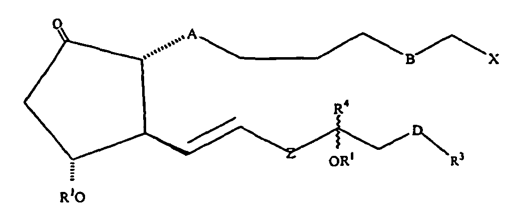

Another preferred group includes compounds having the formula III:

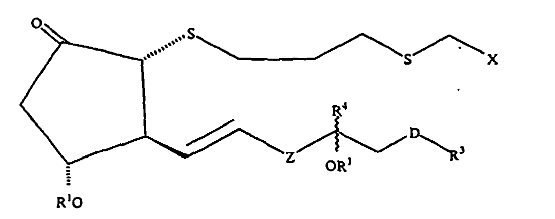

In the above formulae, the substituents and symbols are as hereinabove defined.

In the above formulae:
Preferably A and B are both S.
Preferably D represents a covalent bond or is CH₂; more preferably D is CH₂.
Preferably Z represents a covalent bond.
Preferably R is H.
Preferably R¹ is H.
Preferably, R³ is benzo[b]thienyl or 3-chlorobenzo[b]thienyl.
Preferably R is hydrogen or methyl, most preferably hydrogen.
Preferably Y = O.
Preferably X is CO₂R and more preferably R is selected from the group consisting of H, methyl, i-propyl and n-propenyl.

The above compounds of the present invention may be prepared by methods that are known in the art or according to the working example below. The compounds, below, are especially preferred representative, of the compounds of the present invention.
{3-[(1R,2S,3R)-3-Hydroxy-2-((S)-(E)-3-hydroxy-5-(benzothienyl)pent-1-enyl)-5-oxocyclopentylsulfanyl]propylsulanyl}acetic acid methyl ester,
{3-[(1R,2S,3R)-3-Hydroxy-2-((S)-(E)-3-hydroxy-5-(benzothienyl)pent-1-enyl)-5-oxocyclopentylsulfanyl]propylsulfanyl}acetic acid,
{3-[(1R,2S,3R)-3-Hydroxy-2-((S)-(E)-3-hydroxy-5-(benzothienyl)pent-1-enyl)-5-oxocyclopentylsulfanyl]propylsulfanyl}acetic acid isopropyl ester,
{3-[(1R,2S,3R)-3-Hydroxy-2-((S)-(E)-3-hydroxy-5-(benzofuranyl)pent-1-enyl)-5-oxocyclopentylsulfanyl]propylsulfanyl}acetic acid methyl ester,
{3-[(1R,2S,3R)-3-Hydroxy-2-((S)-(E)-3-hydroxy-5-(benzofuranyl)pent-1-enyl)-5-oxocyclopentylsulfanyl]propylsulfanyl}acetic acid,
{3-[(1R,2S,3R)-3-Hydroxy-2-((S)-(E)-3-hydroxy-5-(benzofuranyl)pent-1-enyl)-5-oxocyclopentylsulfanyl]propylsulfanyl}acetic acid isopropyl ester,
{3-[(1R,2S,3R)-2-((E)-4-Benzo[b]thiophen-3-yl-3-hydroxybut-1-enyl)-3-hydroxy-5-oxocyclopertylsulfanyl]propylsulfanyl}acetic acid methyl ester,
{3-[(1R,2S,3R)-2-((E)-Benzo[b]thiophen-3-yl-3-hydroxybut-1-enyl)-3-hydroxy-5-oxocyclopentylsulfanyl]propylsulfanyl}acetic acid, and
{3-[(1R,2S,3R)-3-Hydroxy-2((S)-(E)-3-(hydroxy)-3-(methyl)-5-(benzothienyl)pent-1-enyl)-5-oxocyclopentylsulfanyl]propylsulfanyl}acetic acid methyl ester.

Pharmaceutical compositions may be prepared by combining a therapeutically effective amount of at least one compound according to the present invention, or a pharmaceutically acceptable acid addition salt thereof, as an active ingredient, with conventional ophthalmically acceptable pharmaceutical excipients, and by preparation of unit dosage forms suitable for topical ocular use. The therapeutically efficient amount typically is between about 0.0001 and about 5% (w/v), preferably about 0.001 to about 1.0% (w/v) in liquid formulations.

For ophthalmic application, preferably solutions are prepared using a physiological saline solution as a major vehicle. The pH of such ophthalmic solutions should preferably be maintained between 6.5 and 7.2 with an appropriate buffer system. The formulations may also contain conventional, pharmaceutically acceptable preservatives, stabilizers and surfactants.

Preferred preservatives that may be used in the pharmaceutical compositions of the present invention include, but are not limited to, benzalkonium chloride, chlorobutanol, thimerosal, phenylmercuric acetate and phenylmercuric nitrate. A preferred surfactant is, for example, Tween 80. Likewise, various preferred vehicles may be used in the ophthalmic preparations of the present invention. These vehicles include, but are not limited to, polyvinyl alcohol, povidone, hydroxypropyl methyl cellulose, poloxamers, carboxymethyl cellulose, hydroxyethyl cellulose and purified water.

Tonicity adjustors may be added as needed or convenient. They include, but are not limited to, salts, particularly sodium chloride, potassium chloride, mannitol and glycerin, or any other suitable ophthalmically acceptable tonicity adjustor.

Various buffers and means for adjusting pH may be used so long as the resulting preparation is ophthalmically acceptable. Accordingly, buffers include acetate buffers, citrate buffers, phosphate buffers and borate buffers. Acids or bases may be used to adjust the pH of these formulations as needed.

In a similar vein, an ophthalmically acceptable antioxidant for use in the present invention includes, but is not limited to, sodium metabisulfite, sodium thiosulfate, acetylcysteine, butylated hydroxyanisole and butylated hydroxytoluene.

Other excipient components which may be included in the ophthalmic preparations are chelating agents. The preferred chelating agent is edentate disodium, although other chelating agents may also be used in place or in conjunction with it.

The ingredients are usually used in the following amounts:

| **Ingredient** | **Amount (% w/v)** |
|---|---|
| active ingredient | about 0.001-5 |
| preservative | 0-0.10 |
| vehicle | 0-40 |
| tonicity adjustor | 1-10 |
| buffer | 0.01-10 |
| pH adjustor | q.s. pH 4.5-7.5 |
| antioxidant | as needed |
| surfactant | as needed |
| purified water | as needed to make 100% |

The actual dose of the active compounds of the present invention depends on the specific compound, and on the condition to be treated; the selection of the appropriate dose is well within the knowledge of the skilled artisan.

The ophthalmic formulations of the present invention are conveniently packaged in forms suitable for metered application, such as in containers equipped with a dropper, to facilitate the application to the eye. Containers suitable for dropwise application are usually made of suitable inert, non-toxic plastic material, and generally contain between about 0.5 and about 15 ml solution.

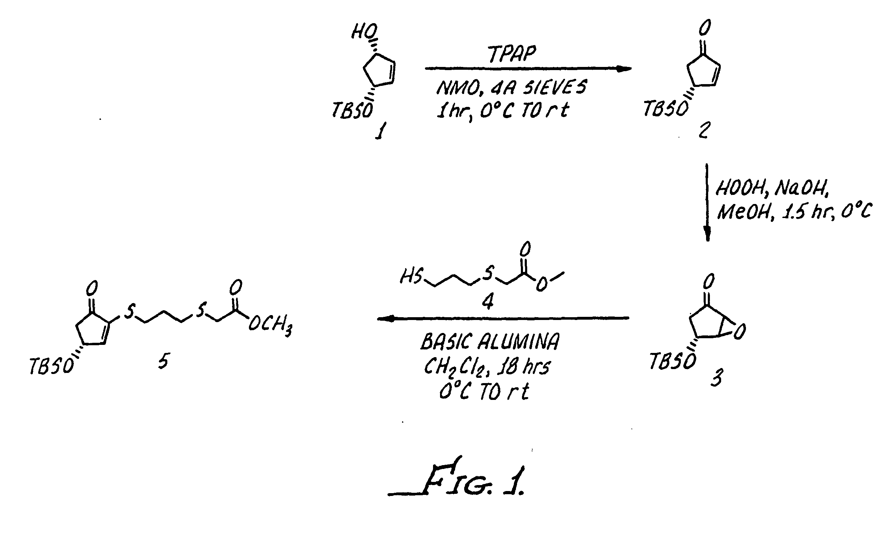

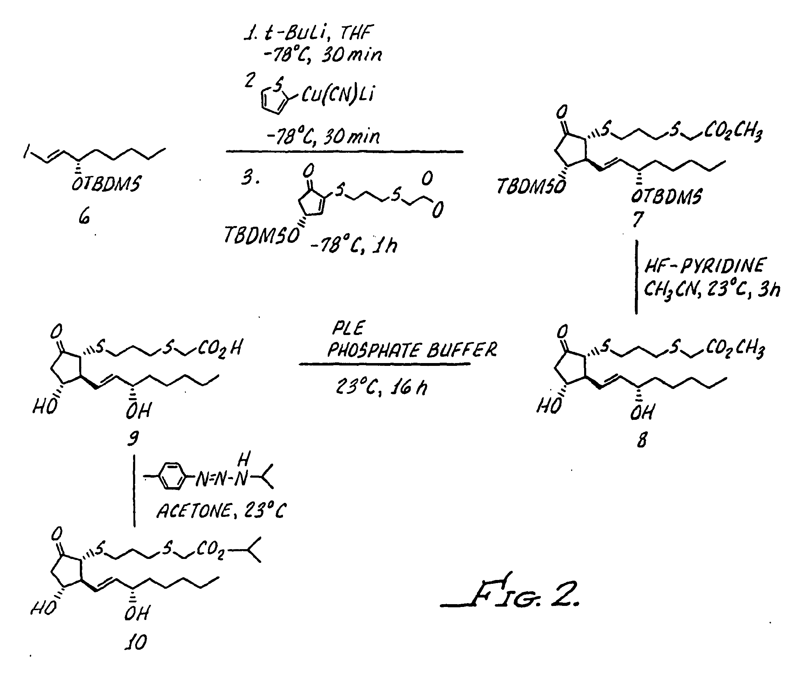

The invention is further illustrated by the following non-limiting Examples, which are summarized in the reaction schemes of Figures 1 and 2 wherein the compounds are identified by the same designator in both the Examples and the Figures.

### Example 1

### (R)-4-(tert-Butyldimethylsilanyloxy)cyclopent-2-enone (2).

Tetrapropylammonium perruthenate (9.4 mg, 0.027 mmol) was added to a mixture of (1S, 4R)-4-(*tert*-butyldimethylsilanyloxy)cyclopent-2-enol prepared, according to Tetrahedron Letters, Vol. 37, No. 18, 1996, pp. 3083-6, (118.6 mg, 0.54 mmol), 4-methylmorpholine N-oxide (94.9 mg, 0.81 mmol) and crushed 4Å sieves (270 mg) in CH₂Cl₂ (10 mL). The mixture was stirred for 30 min and was passed through a plug of silica gel with CH₂Cl₂. The filtrate was concentrated *in vacuo* to give 100 mg (86%) of the above titled compound.

### Example 2

### (R)-4-(tert-Butyldimethylsilanyloxy)-6-oxabicyclo[3.1.0]hexan-2-one (3).

Hydrogen peroxide (4.5 mL, 46.3 mmol, 30% wt. % solution in water) and 1N NaOH (46 µL, 0.046 mmol) were added to a solution of enone 2 (2.5 g, 11.5 mmol) in MeOH (30 mL) at 0° C. After stirring 1.5 h at 0° C the mixture was concentrated *in vacuo*, washed with saturated aqueous NH₄Cl and extracted with CH₂Cl₂ (3X). The combined organics were washed with brine, dried (Na₂SO₄), filtered and concentrated *in vacuo* to afford the above titled compound.

### Example 3

### ({3-[(R)-3-(tert-Butyldimethylsilanyloxy)-5-oxocyclopent-1-enylsulfanyl]propylsulfanyl}acetic acid methyl ester (5).

The epoxide 3 prepared above was diluted with CH₂Cl₂ (30 mL), (3-mercaptopropylsulfanyl) acetic acid methyl ester 4 (1.93 g, 10.7 mmol), prepared according to Chem. Pharm. Bull. 28 (2), 1980, 558-566, was added and the solution was cooled to 0° C. Basic alumina (11.9 g) was added and the reaction mixture was warmed to room temperature. After stirring for 18 h the mixture was filtered through celite and concentrated *in vacuo*. The residue was purified by flash column chromatography (silica gel, 6:1 hex/EtOAc) to yield 3.6 g (80 %) of the above titled compound.

### Example 4

### (3-{(1R,2S,3R)-3-(tert-Butyldimethylsilanyloxy)-2-[(S)-(E)-3-(tert-butyldimethylsilanoxy)oct-1-enyl]-5-oxocyclopentylsulfanyl} propylsulfanyl)acetic acid methyl ester (7).

*tert*-Butyllithium (1.47 mL of a 1.7M solution in pentane, 2.5 mmol) was added dropwise to a solution of *tert*-butyl[(S)-1-((E)-2-iodovinyl) hexyloxy]dimethylsilane 6 (462.5 mg, 1.25 mmol) in Et₂O (6.0 mL) at -78° C. After stirring for 30 min lithium 2-thienylcyanocuprate (6.0 mL of a 0.25M solution in THF, 1.5 mmol) was added and the reaction was stirred an additional 30 min at -78° C. A solution of enone 5 (430 mg, 1.1 mmol) in Et₂O (1 mL) was added and stirring was continued for an additional 1 h. The reaction mixture was then quickly poured into saturated aqueous NH₄Cl cooled to 0° C. The mixture was extracted with EtOAc and the organic portion was washed with brine, dried (Na₂SO₄), filtered and concentrated *in vacuo*. The residue was quickly purified by flash column chromatography (silica gel, 100% hexane followed by 8:1 hex/EtOAc) to afford 270 mg (39%) of the above titled compound.

### Example 5

### {3-[(1R,2S,3R)-3-Hydroxy-2-((S)-(E)-3-hydroxyoct-1-enyl)-5-oxocyclopentylsulfanyl]propylsulfanyl}acetic acid methyl ester (8).

Hydrogen fluoride-pyridine (220 µL) was added to a solution of bis-TBDMS ether 7 (70 mg, 0.11 mmol) in CH₃CN (2.0 mL) at 0° C. The reaction was warmed to room temperature, stirred 1 h, and recooled to 0° C. The reaction was quenched with saturated aqueous NaHCO₃ until gas evolution ceased. The mixture was extracted with CH₂Cl₂ (4X). The combined organics were washed with brine, dried (Na₂SO₄), filtered and concentrated *in vacuo*. Purification of the residue by flash column chromatography (silica gel, 100% CH₂Cl₂ followed by 30:1 CH₂Cl₂:MeOH) provided 40 mg (90%) of the above titled compound.

### Example 6

### {3-[(1R,2S,3R)-3-Hydroxy-2-((S)-(E)-3-hydroxyoct-1-enyl)-5-oxocyclopentylsulfanyl]propylsulfanyl}acetic acid (9).

Methyl ester **8** (50 mg, 0.124 mmol) was dissolved in CH₃CN (10 mL) and pH 7.2 phosphate buffer (3.0 mL) was added. The mixture was treated with PLE (400 µL, 1.34 mol/L) and stirred for 16 h at 23 °C. The reaction mixture was extracted with EtOAc (3X). The combined organics were washed with brine, dried (Na₂SO₄), filtered and concentrated *in vacuo*. Purification of the residue by flash column chromatography (silica gel, 100% EtOAc) gave 5.3 mg (11%) of the above titled compound.

### Example 7

### {3-[(1R,2S,3R)-3-Hydroxy-2-((S)-(E)-3-hydroxyoct-5-enyl)-5-oxocyclopentylsulfanyl]propylsulfanyl}acetic acid isopropyl ester (10).

Isopropyl-*p*-tolyltriazene (200 µL) was added dropwise to a solution of carboxylic acid 9 (10.5 mg, 0.026 mmol) in acetone (5.0 mL) at 23°C. After stirring for 1 h the reaction was quenched with 1N HCl and the solvent was removed *in vacuo*. The residue was extracted with CH₂Cl₂ (2X). The combined organics were dried (Na₂SO₄), filtered and concentrated *in vacuo*. Purification of the residue by flash column chromatography (silica gel, 4:1 hex/EtOAc) gave 4.3 mg (38%) of the above titled compound.

### Example 8 (Reference)

### (3-{(1R,2S,3R)-3-(tert-Butyldimethylsilanyloxy)-2-[(S)-(E)-3-(tert-butyldimethylsilanoxy)-5-(naphthyl)pent-1-enyl]-5-oxocyclopentylsulfanyl} propylsulfanyl)acetic acid methyl ester (H).

### (3-{(1R,2S,3R)-3-(tert-Butyldimethylsilanyloxy)-2-[(S)-(E)-3-(tert-butyldimethylsilanoxy)-5-(naphthyl)pent-1-enyl]-5-oxocyclopentylsulfanyl} propylsulfanyl)acetic acid methyl ester (L).

The named compound is prepared by substituting *tert*-butyl-[(E)-3-iodo-1-(2-naphthalen-2-yl-ethyl)allyloxy]dimethylsilane for *tert*-butyl[(S)-1-((E)-2-iodovinyl)hexyloxy]dimethylsilane in the method of Example 4. FCC gives a higher Rf compound and a lower Rf compound, designated as H and L, respectively.

### Example 9(H) (Reference)

### {3-[(1R,2S,3R)-3-Hydroxy-2-((S)-(E)-3-(hydroxy)-5-(naphthyl)pent-1-enyl)-5-oxocyclopentylsulfanyl]propylsulfanyl}acetic acid methyl ester (H).

The named compound is prepared by repeating the method of Example 5 with the named compound of Example 8 (H) rather than the named compound of Example 4.

### Example 9 (L) (Reference)

### {3-[(1R,2S,3R)-3-Hydroxy-2-((S)-(E)-3-(hydroxy)-5-(naphthyl)pent-1-enyl)-5-oxocyclopentylsulfanyl]propylsulfanyl}acetic acid methyl ester (L).

The named compound is prepared by repeating the method of Example 5 with the named compound of Example 8 (L) rather than the named compound of Example 4.

### Example 10 (H) (Reference)

### {3-[(1R,2S,3R)-3-Hydroxy-2-((S)-(E)-3-(hydroxy)-5-(naphthyl)pent-1-enyl)-5-oxocyclopentylsulfanyl]propylsulfanyl}acetic acid (H).

The named compound is prepared by repeating the method of Example 6 with the named compound of Example 9 (H) rather than the named compound of Example 5.

### Example 10 (L) (Reference)

### {3-[(1R,2S,3R)-3-Hydroxy-2-((S)-(E)-3-(hydroxy)-5-(naphthyl)pent-1-enyl)-5-oxocyclopentylsulfanyl]propylsulfanyl}acetic acid (L).

The named compound is prepared by repeating the method of Example 6 with the named compound of Example 9 (L) rather than the named compound of Example 5.

### Example 11 (Reference)

### {3-[(1R,2S,3R)-3-Hydroxy-2-((S)-(E)-3-(hydroxy)-5-(naphthyl)pent-1-enyl)-5-oxocyclopentylsulfanyl]propylsulfanyl}acetic acid isopropyl ester.

The named compound is prepared by repeating the method of Example 7 with the named compound of Example 10 rather than the named compound of Example 6.

### Example 12

### (3-{(1R,2S,3R)-3-(tert-Butyldimethylsilanyloxy)-2-[(S)-(E)-3-(tert-butyldimethylsilanoxy)-5-(benzothienyl)pent-1-enyl]-5-oxocyclopentylsulfanyl} propylsulfanyl)acetic acid methyl ester (H).

### (3-{(1R,2S,3R)-3-(tert-Butyldimethylsilanyloxy)-2-[(S)-(E)-3-(tert-butyldimethylsilanoxy)-5-(benzothienyl)pent-1-enyl]-5-oxocyclopentylsulfanyl} propylsulfanyl)acetic acid methyl ester (L).

The named compound is prepared by substituting [(E)-1-(2-Benzo[b]thiophen-2-yl-ethyl)-3-iodoallyloxy]-*tert*-butyldimethylsilane for *tert-*butyl[(S)-1-((E)-2-iodovinyl)hexyloxy]dimethylsilane in the method of Example 4. FCC gives a higher Rf compound and a lower Rf compound, designated as H and L, respectively.

### Example 13(H)

### {3-[(1R,2S,3R)-3-Hydroxy-2-((S)-(E)-3-hydroxy-5-(benzothienyl)pent-1-enyl)-5-oxocyclopentylsulfanyl]propylsulfanyl}acetic acid methyl ester (H).

The named compound is prepared by repeating the method of Example 5 with the named compound of Example 12 (H) rather than the named compound of Example 4.

### Example 13(L)

### {3-[(1R,2S,3R)-3-Hydroxy-2-((S)-(E)-3-hydroxy-5-(benzothienyl)pent-1-enyl)-5-oxocyclopentylsulfanyl]propylsulfanyl}acetic acid methyl ester (L).

The named compound is prepared by repeating the method of Example 5 with the named compound of Example 12 (H) rather than the named compound of Example 4.

### Example 14(H)

### {3-[(1R,2S,3R)-3-Hydroxy-2-((S)-(E)-3-hydroxy-5-(benzothienyl)pent-1-enyl)-5-oxocyclopentylsulfanyl]propylsulfanyl}acetic acid (H).

The named compound is prepared by repeating the method of Example 6 with the named compound of Example 13 (H) rather than the named compound of Example 5.

### Example 14(L)

### {3-[(1R,2S,3R)-3-Hydroxy-2-((S)-(E)-3-hydroxy-5-(benzothienyl)pent-1-enyl)-5-oxocyclopentylsulfanyl]propylsulfanyl}acetic acid (L).

The named compound is prepared by repeating the method of Example 6 with the named compound of Example 13 (L) rather than the named compound of Example 5.

### Example 15

### {3-[(1R,2S,3R)-3-Hydroxy-2-((S)-(E)-3-hydroxy-5-(benzothienyl)pent-1-enyl)-5-oxocyclopentylsulfanyl]propylsulfanyl}acetic acid isopropyl ester.

The named compound is prepared by repeating the method of Example 7 with the named compound of Example 14 rather than the named compound of Example 6.

### Example 16

### (3-{(1R,2S,3R)-3-(tert-Butyldimethylsilanyloxy)-2-[(S)-(E)-3-(tert-butyldimethylsilanoxy)-5-(benzofuranyl)pent-1-enyl]-5-oxocyclopentylsulfanyl}propylsulfanyl)acetic acid methyl ester.

The named compound is prepared by substituting [(E)-1-(2-Benzo[*b*]furan-2-yl-ethyl)-3-iodoallyloxy]-*tert*-butyldimethylsilane for *tert*-butyl[(S)-1-((E)-2-iodovinyl) hexyloxy]dimethylsilane in the method of Example 4.

### Example 17

### {3-[(1R,2S,3R)-3-Hydroxy-2-((S)-(E)-3-hydroxy-5-(benzofuranyl)pent-1-enyl)-5-oxocyclopentylsulfanyl]propylsulfanyl}acetic acid methyl ester.

The named compound is prepared by repeating the method of Example 5 with the named compound of Example 16 rather than the named compound of Example 4.

### Example 18

### {3-[(1R,2S,3R)-3-Hydroxy-2-((S)-(E)-3-hydroxy-5-(benzofuranyl)pent-1-enyl)-5-oxocyclopentylsulfanyl]propylsulfanyl}acetic acid.

The named compound is prepared by repeating the method of Example 6 with the named compound of Example 17 rather than the named compound of Example 5.

### Example 19

### {3-[(1R,2S,3R)-3-Hydroxy-2-((S)-(E)-3-hydroxy-5-(benzofuranyl)pent-1-enyl)-5-oxocyclopentylsulfanyl]propylsulfanyl}acetic acid isopropyl ester.

The named compound is prepared by repeating the method of Example 7 with the named compound of Example 18 rather than the named compound of Example 6.

### Example 20 (Reference)

### (3-{(1R,2S,3R)-3-(tert-Butyldimethylsilanyloxy)-2-[(E)-3-(tert-butyldimethylsilanoxy)-4-naphthalen-2-yl-but-1-enyl]-5-oxocyclopentylsulfanyl}propylsulfanyl)acetic acid methyl ester (H).

### (3-{(1R,2S,3R)-3-(tert-Butyldimethylsilanyloxy)-2-[(E)-3-(tert-butyldimethylsilanoxy)-4-naphthalen-2-yl-but-1-enyl]-5-oxocyclopentylsulfanyl} propylsulfanyl)acetic acid methyl ester (L).

The named compound is prepared by substituting *tert*-butyl-((E)-3-iodo-1-naphthalen-2-yl-methylallyloxy)dimethylsilane for *tert*-butyl[(S)-1-((E)-2-iodovinyl) hexyloay]dimethylsilane in the method of Example 4. FCC gives a higher Rf compound and a lower Rf compound, designated as H and L, respectively.

### Example 21 (H) (Reference)

### {3-[(1R,2S,3R)-3-Hydroxy-2-((E)-3-hydroxy-4-naphthalen-2-yl-but-1-enyl)-5-oxocyclopentylsulfanyl]propylsulfanyl}acetic acid methyl ester (H).

The named compound is prepared by repeating the method of Example 5 with the named compound of Example 20 (H) rather than the named compound of Example 4.

### Example 21(L) (Reference)

### {3-[(1R,2S,3R)-3-Hydroxy-2-((E)-3-hydroxy-4-naphthalen-2-yl-but-enyl)-5-oxocyclopentylsulfanyl]propylsulfanyl}acetic acid methyl ester (L).

The named compound is prepared by repeating the method of Example 5 with the named compound of Example 20 (H) rather than the named compound of Example 4.

### Example 22(H) (Reference)

### {3-[(1R,2S,3R)-3-Hydroxy-2-((E)-3-hydroxy-4-naphthalen-2-yl-but-1-enyl)-5-oxocyclopentylsulfanyl]propylsulfanyl}acetic acid (H).

The named compound is prepared by repeating the method of Example 6 with the named compound of Example 21 (H) rather than the named compound of Example 5.

### Example 22(L) (Reference)

### {3-[(1R,2S,3R)-3-Hydroxy-2-((E)-3-hydroxy-4-naphthalen-2-yl-but-1-enyl)-5-oxocyclopentylsulfanyl]propylsulfanyl}acetic acid (L).

The named compound is prepared by repeating the method of Example 6 with the named compound of Example 21 (H) rather than the named compound of Example 5.

### Example 23

### {3-[(1R,2S,3R)-2-[(E)-4-Benzo[b]thiophen-3-yl-3-(tert-butyldimethylsilanyloxy)but-1-enyl]-3-(tert-butyldimethylsilanyloxy)-5-oxocyclopentylsulfanyl]propylsulfanyl}acetic acid methyl ester (H).

### {3-[(1R,2S,3R)-2-[(E)-4-Benzo[b]thiophen-3-yl-3-(tert-butyldimethylsilanyloxy)but-1-enyl]-3-(tert-butyldimethylsilanyloxy)-5-oxocyclopentylsulfanyl]propylsulfanyl}acetic acid methyl ester (L).

The named compound is prepared by substituting ((E)-1-Benzo[*b*]thiophen-3-ylmethyl-3-iodo-allyloxy)-*tert*-butyldimethylsilane for *tert*-butyl[(S)-1-((E)-2-iodovinyl)hexyloxy]dimethylsilane in the method of Example 4. FCC gives a higher Rf compound and a lower Rf compound, designated as H and L respectively.

### Example 24(H)

### {3-[(1R,2S,3R)-2-((E)-4-Benzo[b]thiophen-3-yl-3-hydroxybut-1-enyl)-3-hydroxy-5-oxocyclopentylsulfanyl]propylsulfanyl}acetic acid methyl ester (H).

The named compound is prepared by repeating the method of Example 5 with the named compound of Example 23 (H) rather than the named compound of Example 4.

### Example 24(L)

### {3-[(1R,2S,3R)-2-((E)-4-Benzo[b]thiophen-3-yl-3-hydroxybut-1-enyl)-3-hydroxy-5-oxocyclopentylsulfanyl]propylsulfanyl}acetic acid methyl ester (L).

The named compound is prepared by repeating the method of Example 5 with the named compound of Example 23 (H) rather than the named compound of Example 4.

### Example 25(H)

### {3-[(1R,2S,3R)-2-((E)-4-Benzo[b]thiophen-3-yl-3-hydroxybut-1-enyl)-3-hydroxy-3-oxocyclopentylsulfanyl]propylsulfanyl}acetic acid (H).

The named compound is prepared by repeating the method of Example 6 with the named compound of Example 24 (H) rather than the named compound of Example 5.

### Example 25(L)

### {3-[(1R,2S,3R)-2-((E)-1-Benzo[b]thiophen-3-yl-3-hydroxybut-1-enyl)-3-hydroxy-5-oxocyclopentylsulfanyl]propylsulfanyl}acetic acid (L).

The named compound is prepared by repeating the method of Example 6 with the named compound of Example 24 (H) rather than the named compound of Example 5.

### Example 26 (Reference)

### (3-{(1R,2S,3R)-3-(tert-Butyldimethylsilanyloxy)-2-[(S)-(E)-3-(tert-butyldimethylsilanoxy)-3-(methyl)-5-(naphthyl)pent-1-enyl]-5-oxocyclopentylsulfanyl} propylsulfanyl)acetic acid methyl ester (H).

### (3-{(1R,2S,3R)-3-(tert-Butyldimethylsilanyloxy)-2-[(S)-(E)-3-(tert-butyldimethylsilanoxy)-3-(methyl)-5-(naphthyl)pent-1-enyl]-5-oxocyclopentylsulfanyl} propylsulfanyl)acetic acid methyl ester (L).

The named compound is prepared by substituting *tert*-Butyl-[(E)-3-iodo-1-methyl-1-(2-naphthalen-2-yl-ethyl)allyloxy]dimethylsilane for *tert*-butyl[(S)-1-((E)-2-iodovinyl)hexyloxy]dimethylsilane in the method of Example 4. FCC gives a higher Rf compound and a lower Rf compound, designated as H and L, respectively.

### Example 27(H) (Reference)

### {3-[(1R,2S,3R)-3-Hydroxy-2-((S)-(E)-3-(hydroxy)-3-(methyl)-5-(naphthyl)pent-1-enyl)-5-oxocyclopentylsulfanyl]propylsulfanyl}acetic acid methyl ester (H).

The named compound is prepared by repeating the method of Example 5 with the named compound of Example 26 (H) rather than the named compound of Example 4.

### Example 27(L) (Reference)

### {3-[(1R,2S,3R)-3-Hydroxy-2-((S)-(E)-3-(hydroxy)-3-(methyl)-5-(naphthyl)pent-1-enyl)-5-oxocyclopentylsulfanyl]propylsulfanyl}acetic acid methyl ester (L).

The named compound is prepared by repeating the method of Example 5 with the named compound of Example 26 (H) rather than the named compound of Example 4.

### Example 28(H) (Reference)

### {3-[(1R,2S,3R)-3-Hydroxy-2-((S)-(E)-3-(hydroxy)-3-(methyl)-5-(naphthyl)pent-1-enyl)-5-oxocyclopentylsulfanyl]propylsulfanyl}acetic acid (H).

The named compound is prepared by repeating the method of Example 6 with the named compound of Example 27 (H) rather than the named compound of Example 5.

### Example 28(L) (Reference)

### {3-[(1R,2S,3R)-3-Hydroxy-2-((S)-(E)-3-(hydroxy)-3-(methyl)-5-(naphthyl)pent-1-enyl)-5-oxocyclopentylsulfanyl]propylsulfanyl}acetic acid (L).

The named compound is prepared by repeating the method of Example 6 with the named compound of Example 27(L) rather than the named compound of Example 5.

### Example 29 (Reference)

### (3-{(1R,2S,3R)-3-(tert-Butyldimethylsilanyloxy)-2-[(E)-3-(tert-butyldimethylsilanoxy)-3-methyl-4-naphthalen-2-yl-but-1-enyl]-5-oxocyclopentylsulfanyl} propylsulfanyl)acetic acid methyl ester (H).

### (3-{(1R,2S,3R)-3-(tert-Butyldimethylsilanyloxy)-2-[(E)-3-(tert-butyldimethylsilanoxy)-3-methyl-4-naphthalen-2-yl-but-1-enyl]-5-oxocyclopentylsulfanyl} propylsulfanyl)acetic acid methyl ester (L).

The named compound is prepared by substituting *tert*-butyl-[(E)-3-iodo-1-methyl-1-(2-naphthalen-2-yl-methyl)allyloay]dimethylsilane for *tert*-butyl[(S)-1-((E)-2-iodovinyl) hexyloxy]dimethylsilane in the method of Example 4. FCC gives a higher Rf compound and a lower Rf compound, designated as H and L, respectively.

### Example 30(H) (Reference)

### {3-[(1R,2S,3R)-3-Hydroxy-2-((E)-3-hydroxy-3-methyl-4-naphthalen-2-yl-but-1-enyl)-5-oxocyclopentylsulfanyl]propylsulfanyl}acetic acid methyl ester (H).

The named compound is prepared by repeating the method of Example 5 with the named compound of Example 29 (H) rather than the named compound of Example 4.

### Example 30(L) (Reference)

### {3-[(1R,2S,3R)-3-Hydroxy-2-((E)-3-hydroxy-3-methyl-4-naphthalen-2-yl-but-1-enyl)-5-oxocyclopentylsulfanyl]propylsulfanyl}acetic acid methyl ester (L).

The named compound is prepared by repeating the method of Example 5 with the named compound of Example 29 (L) rather than the named compound of Example 4.

### Example 31(H) (Reference)

### {3-[(1R,2S,3R)-3-Hydroxy-2-((E)-3-hydroxy-3-methyl-4-naphthalen-2-yl-but-1-enyl)-5-oxocyclopentylsulfanyl]propylsulfanyl}acetic acid (H).

The named compound is prepared by repeating the method of Example 6 with the named compound of Example 30 (H) rather than the named compound of Example 5.

### Example 31(L) (Reference)

### {3-[(1R,2S,3R)-3-Hydroxy-2-((E)-3-hydroxy-3-methyl-4-naphthalen-2-yl-but-1-enyl)-5-oxocyclopentylsulfanyl]propylsulfanyl}acetic acid (L).

The named compound is prepared by repeating the method of Example 6 with the named compound of Example 30 (L) rather than the named compound of Example 5.

### Example 32

### (3-{(1R,2S,3R)-3-(tert-Butyldimethylsilanyloxy)-2-[(S)-(E)-3-(tert-butyldimethylsilanoxy)-3-(methyl)-5-(benzylthienyl)pent-1-enyl]-5-oxocyclopentylsulfanyl} propylsulfanyl)acetic acid methyl ester (H).

### (3-{(1R,2S,3R)-3-(tert-Butyldimethylsilanyloxy)-2-[(S)-(E)-3-(tert-butyldimethylsilanoxy)-3-(methyl)-5-(benzothienyl)pent-1-enyl]-5-oxocyclopentylsulfanyl} propylsulfanyl)acetic acid methyl ester (L).

The named compound is prepared by [(E)-1-(2-Benzo[*b*]thiophen-2-yl-ethyl)-3-iodo-1-methylallyloxy]-*tert*-butyldimethylsilane for *tert*-butyl[(S)-1-((E)-2-iodovinyl) hexyloxy]dimethylsilane in the method of Example 4. FCC gives a higher Rf compound and a lower Rf compound, designated as H and L, respectively.

### Example 33(H)

### {3-[(1R,2S,3R)-3-Hydroxy-2-((S)-(E)-3-(hydroxy)-3-(methyl)-5-(benzothienyl)pent-1-enyl)-5-oxocyclopentylsulfanyl]propylsulfanyl}acetic acid methyl ester (H).

The named compound is prepared by repeating the method of Example 5 with the named compound of Example 32 (H) rather than the named compound of Example 4.

### Example 33(L)

### {3-[(1R,2S,3R)-3-Hydroxy-2-((S)-(E)-3-(hydroxy)-3-(methyl)-5-(benzothienyl)pent-1-enyl)-5-oxocyclopentylsulfanyl]propylsulfanyl}acetic acid methyl ester (L).

The named compound is prepared by repeating the method of Example 5 with the named compound of Example 32 (L) rather than the named compound of Example 4.

### Example 34(H)

### {3-[(1R,2S,3R)-3-Hydroxy-2-((S)-(E)-3-(hydroxy)-3-(methyl)-5-(benzothienyl)pent-1-enyl)-5-oxocyclopentylsulfanyl]propylsulfanyl}acetie acid (H).

The named compound is prepared by repeating the method of Example 6 with the named compound of Example 33 (H) rather than the named compound of Example 5.

### Example 34(L)

### {3-[(1R,2S,3R)-3-Hydroxy-2-((S)-(E)-3-(hydroxy)-3-(methyl)-5-(benzothienyl)pent-1-enyl)-3-oxocyclopentylsulfanyl]propylsulfanyl}acetic acid (L).

The named compound is prepared by repeating the method of Example 6 with the named compound of Example 33L rather than the named compound of Example 5.

The effects of the compounds of this invention on intraocular pressure may be measured as follows. The compounds are prepared at the desired concentrations in a vehicle comprising 0.1% polysorbate 80 and 10 mM TRIS base. Dogs are treated by administering 25 µl to the ocular surface, the contralateral eye receives vehicle as a control. Intraocular pressure is measured by applanation pneumatonometry. Dog intraocular pressure is measured immediately before drug administration and at 6 hours thereafter.

The compounds of Examples 9H, 9L, 10H, 10L, 13H, 13L, 14H, 14L, 21H, 21L, 22H, 22L, 24H, 25H, 25L, 27H, 27L, 28H, 28L, 30H, 30L, 31H, 31L, 33H, 33L, 34H and 34L are useful in lowering elevated intraocular pressure in mammals, e.g. humans.

The compounds of the Examples are subject to *in vitro* testing as described below. The results are reported in the table.

| Example No. | | hEP₂ | hEP₃ | hEP₄ |
|---|---|---|---|---|
| 33H | 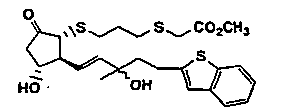 | NA | NA | 200 |
| 33L | 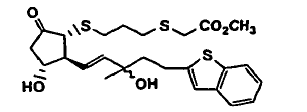 | NA | NA | 300 |
| 34H | 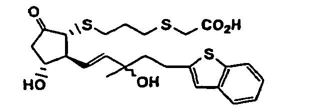 | >>10⁴ | >10⁴ | 32 |
| 34L | 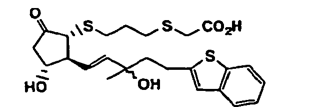 | NA | >10⁴ | 68 |
| 13H | 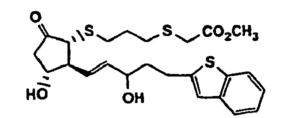 | NA | NA | 91 |
| 13L | 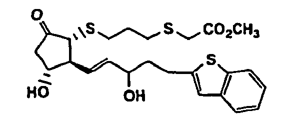 | >>10⁴ | 7200 | 93 |
| 14H | 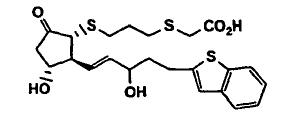 | >>10⁴ | >10⁴ | 27 |
| 14L | 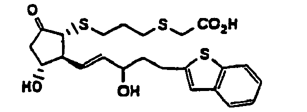 | 10⁴ | >10⁴ | 13 |
| 9H (Ref.) | 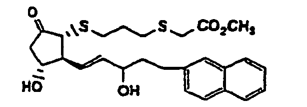 | NA | NA | 40 |
| 9L (Ref.) | 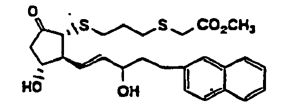 | NA | >10⁴ | 40 |
| 10H (Ref.) | 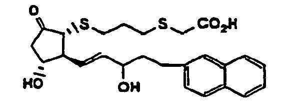 | >>10⁴ | >10⁴ | 450 |
| 10L (Ref.) | 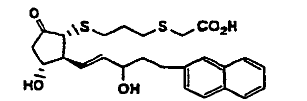 | >10⁴ | 8300 | 19.5 |
| 27H (Ref.) | 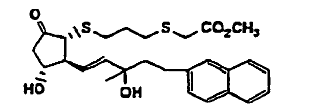 | NA | NA | 500 |
| 27L (Ref.) | 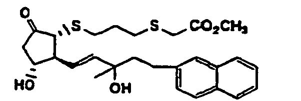 | NA | NA | 3400 |
| 28H (Ref.) | 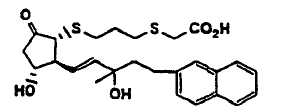 | NA | >10⁴ | 1700 |
| 28L (Ref.) | 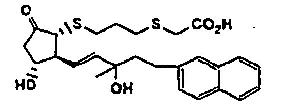 | NA | >10⁴ | 1500 |
| 21H (Ref.) | 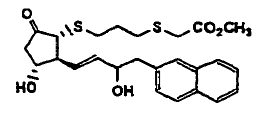 | NA | >10⁴ | 100 |
| | | | | |
| 21L (Ref.) | 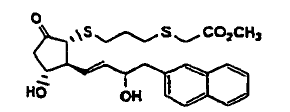 | NA | >10⁴ | 13 |
| 22H (Ref.) | 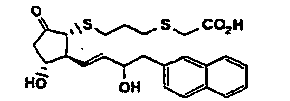 | NA | >10⁴ | 32 |
| 22L (Ref.) | 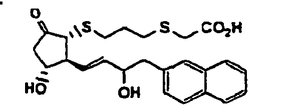 | >>10⁴ | >10⁴ | 6.2 |
| 30H (Ref.) | 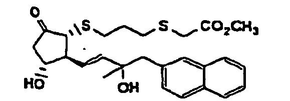 | NA | >10⁴ | 3100 |
| 30L (Ref.) | 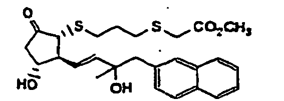 | NA | NA | 3200 |
| 31H (Ref.) | 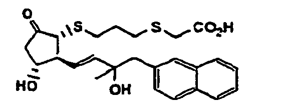 | NA | 8100 | 300 |
| 31L (Ref.) | 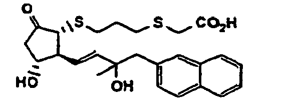 | NA | 9300 | 900 |
| 24H | 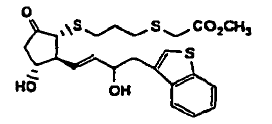 | NA | NA | 200 |
| 24L | 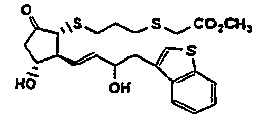 | 9300 | >10⁴ | 30 |
| 25H | 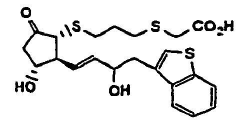 | >10⁴ | NA | 69 |
| | | | | |
| 25L | 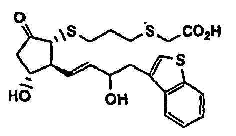 | 2200 | >10⁴ | 5 |

| | | | | |
|---|---|---|---|---|
| "Ref." = Reference Example, not in accordance with the invention | | | | |

### HUMAN RECOMBINANT EP₁, EP₂, EP₄, AND FP RECEPTORS: STABLE TRANSFECTANTS.

Plasmids encoding the human EP₁, EP₂, EP₄, and FP receptors were prepared by cloning the respective coding sequences into the eukaryotic expression vector pCEP4 (Invitrogen). The pCEP4 vector contains an Epstein Barr virus (EBV) origin of replication, which permits episomal replication in primate cell lines expressing EBV nuclear antigen (EBNA-1). It also contains a hygromycin resistance gene that is used for eukaryotic selection. The cells employed for stable transfection were human embryonic kidney cells (HEK-293) that were transfected with and express the EBNA-1 protein. These HEK-293-EBNA cells (Invitrogen) were grown in medium containing Geneticin (G418) to maintain expression of the EBNA-1 protein. HEK-293 cells were grown in DMEM with 10% fetal bovine serum (FBS), 250 µg ml⁻¹ G418 (Life Technologies) and 200 µg ml⁻¹ gentamicin or penicillin/streptomycin. Selection of stable transfectants was achieved with 200µg ml⁻¹ hygromycin, the optimal concentration being determined by previous hygromycin kill curve studies.

For transfection, the cells were grown to 50-60% confluency on 10 cm plates. The plasmid pCEP4 incorporating cDNA inserts for the respective human prostanoid receptor (20 µg) was added to 500 µl of 250 mM CaCl₂. HEPES buffered saline x 2 (2 x HBS, 280 mM NaCl, 20 mM HEPES acid, 1.5 mM Na₂ HPO₄, pH 7.05 - 7.12) was then added dropwise to a total of 500 µl, with continuous vortexing at room temperature. After 30 min, 9 ml DMEM were added to the mixture. The DNA/DMEM/calcium phosphate mixture was then added to the cells, which had been previously rinsed with 10 ml PBS. The cells were then incubated for 5 hr at 37° C in humidified 95% air/5% CO₂. The calcium phosphate solution was then removed and the cells were treated with 10% glycerol in DMEM for 2 min. The glycerol solution was then replaced by DMEM with 10% FBS. The cells were incubated overnight and the medium was replaced by DMEM/10% FBS containing 250 µg ml⁻¹ G418 and penicillin/streptomycin. The following day hygromycin B was added to a final concentration of 200 µg ml⁻¹.

Ten days after transfection, hygromycin B resistant clones were individually selected and transferred to a separate well on a 24 well plate. At confluence each clone was transferred to one well of a 6 well plate, and then expanded in a 10 cm dish. Cells were maintained under continuous hygromycin selection until use.

### HUMAN RECOMBINANT EP₃ AND TP RECEPTORS: TRANSIENT TRANSFECTANTS.

Plasmids encoding the human EP₃ (D isoform) or TP receptor were prepared by cloning the respective coding sequences into a pcDNA₃ vector (Invitrogen). COS-7 cells were transfected with pcDNA₃ containing cDNA encoding the EP₃ or TP receptor by employing the lipofectin method, according to the manufacturers instructions (Gibco). For radioligand binding studies, cells were harvested two days after transfection.

### RADIOLIGAND BINDING

Radioligand binding studies on plasma membrane fractions prepared for cells stably transfected with the cat or human receptor were performed as follows. Cells washed with TME buffer were scraped from the bottom of the flasks and homogenized for 30 sec using a Brinkman PT 10/35 polytron. TME buffer was added as necessary to achieve a 40 ml volume in the centrifuge tubes. TME is comprised of 50 mM TRIS base, 10 mM MgCl₂, 1mM EDTA; pH 7.4 is achieved by adding 1 N HCl. The cell homogenate was centrifuged at 19,000 rpm for 20-25 min at 4°C using a Beckman Ti-60 or Ti-70 rotor. The pellet was then resuspended in TME buffer to provide a final protein concentration of 1 mg/ml, as determined by Bio-Rad assay. Radioligand binding assays were performed in a 100 µl or 200 µl volume.

The binding of [³H](N) PGE₂ (specific activity 165 Ci/mmol) was determined in duplicate and in at least 3 separate experiments. Incubations were for 60 min at 25° C and were terminated by the addition of 4 ml of ice-cold 50 mM TRIS-HCl followed by rapid filtration through Whatman GF/B filters and three additional 4 ml washes in a cell harvester (Brandel). Competition studies were performed using a final concentration of 2.5 or 5 nM [³H](N) PGE₂ and non-specific binding was determined with 10⁻⁵ M unlabelled PGE₂.

For radioligand binding on the transient transfectants, plasma membrane fraction preparation was as follows. COS-7 cells were washed with TME buffer, scraped from the bottom of the flasks, and homogenized for 30 sec using a Brinkman PT 10/35 polytron. TME buffer was added to achieve a final 40 ml volume in the centrifuge tubes. The composition of TME is 100 mM TRIS base, 20 mM MgCl₂, 2M EDTA; 10N HCl is added to achieve a pH of 7.4.

The cell homogenate was centrifuged at 19000 rpm for 20 min at 4°C using a Beckman Ti-60 rotor. The resultant pellet was resuspended in TME buffer to give a final 1 mg/ml protein concentration, as determined by Biorad assay. Radioligand binding assays were performed in a 200 µl volume.

The binding of [³H] PGE₂ (specific activity 165 Ci or mmol ⁻¹) at EP_{3D}, receptors and [³H]-SQ29548 (specific activity 41.5 Ci mmol⁻¹) at TP receptors were determined in duplicate in at least three separate experiments. Radiolabeled PGE₂ was purchased from Amersham, radiolabeled SQ29548 was purchased from New England Nuclear. Incubations were for 60 min at 25°C and were terminated by the addition of 4 ml of ice-cold 50 mM TRIS-HCl, followed by rapid filtration through Whatman GF/B filters and three additional 4 ml washes in a cell harvester (Brandel). Competition studies were performed using a final concentration of 2.5 or 5 nM [³H]-PGE₂, or 10 nM [³H]-SQ 29548 and non-specific binding determined with 10 µM of the respective unlabeled prostanoid. For all radioligand binding studies, the criteria for inclusion were >50% specific binding and between 500 and 1000 displaceable counts or better.

## Claims

1. Use of a compound represented by the general formula I; 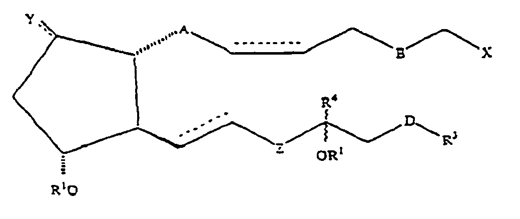 wherein hatched lines represent the α configuration, a triangle represents the β configuration, a wavy line represents either the α configuration or the β configuration and a dotted line represents the presence or absence of a double bond; A and B are independently selected from the group consisting of O, S and CH₂, provided that at least one of A or B is S;
D represents a covalent bond or CH₂, O, S or NH;
X is CO₂R, CONR₂, CH₂OR, P(O)(OR)₂, CONRSO₂R, SONR₂ or 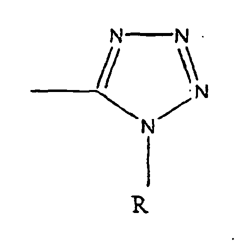 Y is O, OH, OCOR², halogen or cyano;
Z is CH₂ or a covalent bond;
R is H or R²;
R¹ is H, R² phenyl, or COR²;
R² is C₁-C₅ lower alkyl or alkenyl;
R₃ is benzothienyl, benzofuranyl, or substituted derivatives thereof,
wherein the substituents maybe selected from the group consisting of C₁-C₅ alkyl, halogen, CF₃, CN, NO₂, NR₂, CO₂R and OR and R⁴ is hydrogen or C₁-C₅ lower alkyl, for the manufacture of a medicament for the treatment of ocular hypertension or glaucoma.

2. The use according to claim 1 wherein said compound is represented by the general formula II;

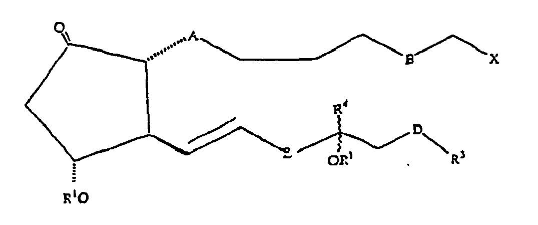

3. The use according to claim 2 wherein said compound is represented by the general formula III;

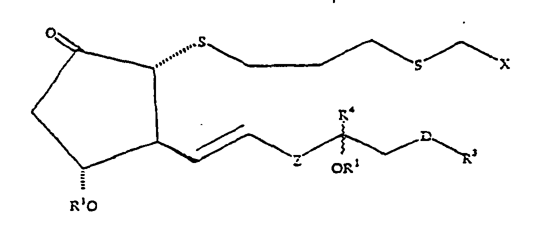

4. The use of claim 1 wherein Z represents a covalent bond.

5. The use of claim 1 wherein D represents a covalent bond or is CH₂.

6. The use of claim 1 wherein X is CO₂ R.

7. The use of claim 6 wherein R is selected from the group consisting of H, methyl, i-propyl, and n-propenyl.

8. The use of claim 1 wherein R is H, or n-propenyl.

9. The use of claim 1 wherein R₁ is H.

10. The use of claim 1 wherein D is CH₂.

11. The use of claim 10 wherein R³ is benzo[b]thienyl or 3-chlorobenzo[b]thienyl.

12. The use of claim 1 wherein said compound is selected from the group consisting of
{3-[(1R,2S,3R)-3-Hydroxy-2-((S)-(E)-3-hydroxy-5-(benzothienyl)pent-1-enyl)-5-oxocyclopentylsulfanyl]propylsulfanyl}acetic acid methyl ester,
{3-[(1R,2S,3R)-3-Hydroxy-2-((S)-(E)-3-hydroxy-5-(benzothienyl)pent-1-enyl)-5-oxocyclopentylsulfanyl]propylsulfanyl}acetic acid,
{3-[(1R,2S,3R)-3-Hydroxy-2-((S)-(E)-3-hydroxy-5-(benzothienyl)pent-1-enyl)-5-oxocyclopentylsulfanyl]propylsulfanyl}acetic add isopropyl ester,
{3-[(1R,2S,3R)-3-Hydroxy-2-((S)-(E)-3-hydroxy-5-(benzofuranyl)pent-1-enyl)-5-oxocyclopentylsulfanyl]propylsulfanyl}acetic acid methyl ester,
{3-[(1R,2S,3R)-3-Hydroxy-2-((S)-(E)-3-hydroxy-5-(benzofuranyl)pent-1-enyl)-5-oxocyclopentylsulfanyl]propylsulfanyl}acetic acid,
{3-[(1R,2S,3R)-3-Hydroxy-2-((S)-(E)-3-hydroxy-5-(benzofuranyl)pent-1-enyl)-5-oxocyclopentylsulfanyl]propylsulfanyl}acetic acid isopropyl ester,
{3-[(1R,2S,3R)-2-((E)-4-Benzo[b]thiophen-3-yl-3-hydroxybut-1-enyl)-3-hydroxy-5-oxocyclopentylsulfanyl]propylsulfanyl}acetic acid methyl easter,
{3-[(1R,2S,3R)-2-((E)-4-Benzo[b]thiophen-3-yl-3-hydroxybut-1-enyl)-3-hydroxy-5-oxocyclopentylsulfanyl]propylsulfanyl}acetic acid, and
{3-[(1R,2S,3R)-3-Hydroxy-2-((S)-(E)-3-(hydroxy)-3-(methyl)-5-(benzothienyl)pent-1-enyl)-5-oxocyclopentylsulfanyl]propylsulfanyl}acetic acid methyl ester.

13. The use of claim 1 wherein D is CH₂ and Z represents a covalent bond.

14. The use of claim 1 wherein R⁴ is hydrogen or methyl.

15. The use of claim 1 wherein R⁴ is hydrogen.

16. An ophthalmic solution comprising a therapeutically effective amount of a compound represented by the general Formula 1 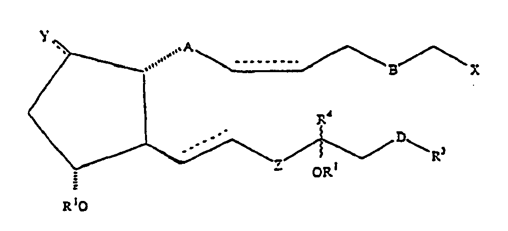 wherein hatched lines represent the α configuration, a triangle represents the β configuration, a wavy line represents the α configuration or the β configuration and a dotted line represents the presence or absence of a double bond;
A and B are independently selected from the group consisting of O, S and CH₂; provided that at least one of A or B is S;
D represents a covalent bond or CH₂ , O, S or NH;
X is CO₂R, CONR₂, CH₂OR, P(O)(OR)₂, CONRSO₂R, SONR₂ or 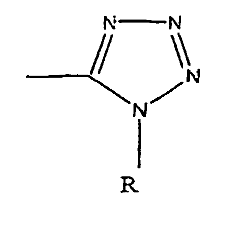 Y is O, OH, OCOR², halogen or cyano;
Z is CH₂ or a covalent bond;
R is H or R²;
R¹ is H, R² , phenyl, or COR²;
R² is C₁-C₅ lower alkyl or alkenyl;
R₃ is benzothienyl, benzofuranyl or substituted derivatives thereof,
wherein the substituents maybe selected from the group consisting of C₁-C₅ alkyl, halogen, CF₃, CN, NO₂, NR₂, CO₂R and OR and R⁴ is hydrogen or C₁-C₅ alkyl, in admixture with a non-toxic, ophthalmically acceptable liquid vehicle, packaged in a container suitable for metered application, wherein the pH of the ophthalmic solution is maintained between 6.5 and 7.2 with an appropriate buffer solution.

17. A pharmaceutical product, comprising a container adapted to dispense the contents of said container in metered form; and an ophthalmic solution according to claim 16 in said container.

18. A novel compound selected from the group consisting of
{3-[(1R,2S,3R)-3-Hydroxy-2-((S)-(E)-3-hydroxy-5-(benzothienyl)pent-1-enyl)-5-oxocyclopentylsulfanyl]propylsulfanyl}acetic add methyl ester,
{3-[(1R,2S,3R)-3-Hydroxy-2-((S)-(E)-3-hydroxy-5-(benzothienyl)pent-1-enyl)-5-oxocyclopentylsulfanyl]propylsulfanyl}acetic acid,
{3-[(1R,2S,3R)-3-Hydroxy-2-((S)-(E)-3-hydroxy-5-(benzothienyl)pent-1-enyl)-5-oxocyclopentylsulfanyl]propylsulfanyl}acetic acid isopropyl ester,
{3-[(1R,2S,3R)-3-Hydroxy-2-((S)-(E)-3-hydroxy-5-(benzofuranyl)pent-1-enyl)-5-oxocyclopentylsulfanyl]propylsulfanyl}acetic acid methyl ester,
{3-[(1R,2S,3R)-3-Hydroxy-2-((S)-(E)-3-hydroxy-5-(benzofuranyl)pent-1-enyl)-5-oxocyclopentylsulfanyl]propylsulfanyl}acetic acid,
{3-[(1R,2S,3R)-3-Hydroxy-2-((S)-(E)-3-hydroxy-5-(benzofuranyl)pent-1-enyl)-5-oxocyclopentylsulfanyl]propylsulfanyl}acetic acid isopropyl ester,
{3-[(1R,2S,3R)-2-((E)-4-Benzo[b]thiophen-3-yl-3-hydroxybut-1-enyl)-3-hydroxy-5-oxocyclopentylsulfanyl]propylsulfanyl}acetic acid methyl ester,
{3-[(1R,2S,3R)-2-((E)-4-Benzo[b]thiophen-3-yl-3-hydroxybut-1-enyl)-3-hydroxy-5-oxocyclopentylsulfanyl]propylsulfanyl}acetic acid, and
{3-[(1R,2S,3R)-3-Hydroxy-2-((S)-(E)-3-(hydroxy)-3-(methyl)-5-(benzothienyl)pent-1-enyl)-5-oxocyclopentylsulfanyl]propylsulfanyl}acetic acid methyl ester.

## Patentansprüche

1. Verwendung einer Verbindung der allgemeinen Formel (I) 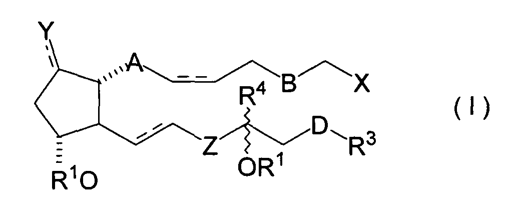 worin
schraffierte Linien die α-Konfiguration darstellen, ein Dreieck die β-Konfiguration darstellt, eine Wellenlinie entweder die α-Konfiguration oder die β-Konfiguration darstellt und eine gepunktete Linie die Gegenwart oder Abwesenheit einer Doppelbindung darstellt;
A und B unabhängig voneinander ausgewählt sind aus der Gruppe bestehend aus O, S und CH₂, vorausgesetzt, dass zumindest eines von A oder B S ist;
D eine kovalente Bindung oder CH₂, O, S oder NH darstellt;
X CO₂R, CONR₂, CH₂OR, P(O)(OR)₂, CONRSO₂R, SONR₂ oder 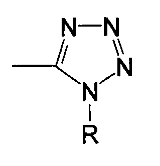 ist;
Y O, OH, OCOR², Halogen oder Cyano ist;
Z CH₂ oder eine kovalente Bindung ist;
R H oder R² ist;
R¹ H, R², Phenyl oder COR² ist;
R² C₁₋₅-Niederalkyl oder Alkenyl ist;
R³ Benzothienyl, Benzofuranyl oder substituierte Derivate davon ist, worin die Substituenten aus der Gruppe bestehend aus C₁₋₅-Alkyl, Halogen, CF₃, CN, NO₂, NR₂, CO₂R und OR ausgewählt sein können und R⁴ Wasserstoff oder C₁₋₅-Niederalkyl ist,
zur Herstellung eines Medikaments für die Behandlung von okularem Bluthochdruck oder Glaukom.

2. Verwendung gemäss Anspruch 1, worin die Verbindung durch die allgemeine Formel (II) dargestellt ist:

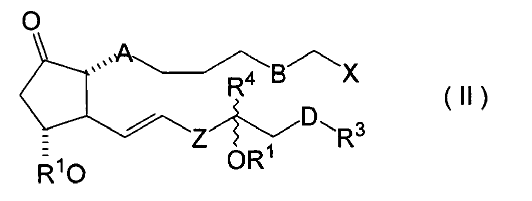

3. Verwendung gemäss Anspruch 2, worin die Verbindung durch die allgemeine Formel (III) dargestellt ist:

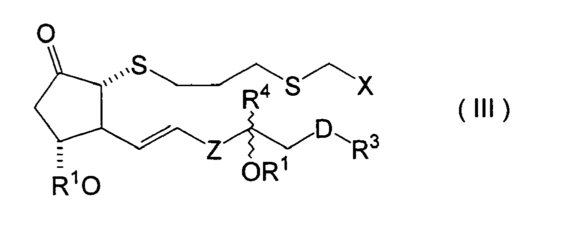

4. Verwendung gemäss Anspruch 1, worin Z eine kovalente Bindung darstellt.

5. Verwendung gemäss Anspruch 1, worin D eine kovalente Bindung darstellt oder CH₂ ist.

6. Verwendung gemäss Anspruch 1, worin X CO₂R ist.

7. Verwendung gemäss Anspruch 6, worin R ausgewählt ist aus der Gruppe bestehend aus H, Methyl, i-Propyl und n-Propenyl.

8. Verwendung gemäss Anspruch 1, worin R H oder n-Propenyl ist.

9. Verwendung gemäss Anspruch 1, worin R₁ H ist.

10. Verwendung gemäss Anspruch 1, worin D CH₂ ist.

11. Verwendung gemäss Anspruch 10, worin R³ Benzo[b]thienyl oder 3-Chlorbenzo[b]thienyl ist.

12. Verwendung gemäss Anspruch 1, worin die Verbindung ausgewählt ist aus der Gruppe bestehend aus:
{3-[(1R,2S,3R)-3-Hydroxy-2-((S)-(E)-3-hydroxy-5-(benzothienyl)pent-1-enyl)-5-oxocyclopentylsulfanyl]-propylsulfanyl}essigsäuremethylester,
{3-[(1R,2S,3R)-3-Hydroxy-2-((S)-(E)-3-hydroxy-5-(benzothienyl)pent-1-enyl)-5-oxocyclopentylsulfanyl]-propylsulfanyl}essigsäure,
{3-[(1R,2S,3R)-3-Hydroxy-2-((S)-(E)-3-hydroxy-5-(benzothienyl)pent-1-enyl)-5-oxocyclopentylsulfanyl]-propylsulfanyl}essigsäureisopropylester,
{3-[(1R,2S,3R)-3-Hydroxy-2-((S)-(E)-3-hydroxy-5-(benzofuranyl)pent-1-enyl)-5-oxocyclopentylsulfanyl]-propylsulfanyl}essigsäuremethylester,
{3-[(1R,2S,3R)-3-Hydroxy-2-((S)-(E)-3-hydroxy-5-(benzofuranyl)pent-1-enyl)-5-oxocyclopentylsulfanyl]-propylsulfanyl}essigsäure,
{3-[(1R,2S,3R)-3-Hydroxy-2-((S)-(E)-3-hydroxy-5-(benzofuranyl)pent-1-enyl)-5-oxocyclopentylsulfanyl]-propylsulfanyl}essigsäureisopropylester,
{3-[(1R,2S,3R)-2-((E)-4-Benzo[b]thiophen-3-yl-3-hydroxybut-1-enyl)-3-hydroxy-5-oxocyclopentylsulfanyl]propylsulfanyl}essigsäuremethylester,
{3-[(1R,2S,3R)-2-((E)-4-Benzo[b]thiophen-3-yl-3-hydroxybut-1-enyl)-3-hydroxy-5-oxocyclopentylsulfanyl]propylsulfanyl}essigsäure und
{3-[(1R,2S,3R)-3-Hydroxy-2-((S)-(E)-3-(hydroxy)-3-(methyl)-5-(benzothienyl)pent-1-enyl)-5-oxocyclopentylsulfanyl]propylsulfanyl}essigsäuremethylester.

13. Verwendung gemäss Anspruch 1, worin D CH₂ ist und Z eine kovalente Bindung darstellt.

14. Verwendung gemäss Anspruch 1, worin R⁴ Wasserstoff oder Methyl ist.

15. Verwendung gemäss Anspruch 1, worin R⁴ Wasserstoff ist.

16. Ophthalmische Lösung, umfassend eine therapeutisch wirksame Menge einer Verbindung der allgemeinen Formel (I): 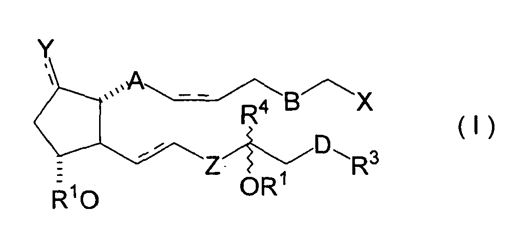 worin
schraffierte Linien die α-Konfiguration darstellen, ein Dreieck die β-Konfiguration darstellt, eine Wellenlinie entweder die α-Konfiguration oder die β-Konfiguration darstellt und eine gepunktete Linie die Gegenwart oder Abwesenheit einer Doppelbindung darstellt;
A und B unabhängig voneinander ausgewählt sind aus der Gruppe bestehend aus O, S und CH₂, vorausgesetzt, dass zumindest eines von A oder B S ist;
D eine kovalente Bindung oder CH₂, O, S oder NH darstellt;
X CO₂R, CONR₂, CH₂OR, P(O)(OR)₂, CONRSO₂R, SONR₂ oder 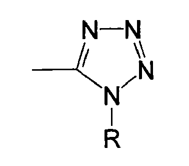 ist;
Y O, OH, OCOR², Halogen oder Cyano ist;
Z CH₂ oder eine kovalente Bindung ist;
R H oder R² ist;
R¹ H, R², Phenyl oder COR² ist;
R² C₁₋₅-Niederalkyl oder Alkenyl ist;
R³ Benzothienyl, Benzofuranyl oder substituierte Derivate davon ist, worin die Substituenten aus der Gruppe bestehend aus C₁₋₅-Alkyl, Halogen, CF₃, CN, NO₂, NR₂, CO₂R und OR ausgewählt sein können und R⁴ Wasserstoff oder C₁₋₅-Niederalkyl ist,
in Mischung mit einem nicht-toxischen, ophthalmisch annehmbaren, flüssigen Träger, abgepackt in einem für eine abgemessene Verabreichung geeigneten Behälter, wobei der pH-Wert der ophthalmischen Lösung durch eine geeignete Pufferlösung zwischen 6,5 und 7,2 gehalten wird.

17. Pharmazeutisches Produkt, umfassend einen Behälter, der so angepasst ist, um den fertigen Inhalt des Behälters in abgemessener Form abzugeben; und eine ophthalmische Lösung gemäss Anspruch 16 in diesem Behälter.

18. Neue Verbindung, ausgewählt aus der Gruppe bestehend aus:
{3-[(1R,2S,3R)-3-Hydroxy-2-((S)-(E)-3-hydroxy-5-(benzothienyl)pent-1-enyl)-5-oxocyclopentylsulfanyl]-propylsulfanyl}essigsäuremethylester,
{3-[(1R,2S,3R)-3-Hydroxy-2-((S)-(E)-3-hydroxy-5-(benzothienyl)pent-1-enyl)-5-oxocyclopentylsulfanyl]-propylsulfanyl}essigsäure,
{3-[(1R,2S,3R)-3-Hydroxy-2-((S)-(E)-3-hydroxy-5-(benzothienyl)pent-1-enyl)-5-oxocyclopentylsulfanyl]-propylsulfanyl}essigsäureisopropylester,
{3-[(1R,2S,3R)-3-Hydroxy-2-((S)-(E)-3-hydroxy-5-(benzofuranyl)pent-1-enyl)-5-oxocyclopentylsulfanyl]-propylsulfanyl}essigsäuremethylester,
{3-[(1R,2S,3R)-3-Hydroxy-2-((S)-(E)-3-hydroxy-5-(benzofuranyl)pent-1-enyl)-5-oxocyclopentylsulfanyl]-propylsulfanyl}essigsäure,
{3-[(1R,2S,3R)-3-Hydroxy-2-((S)-(E)-3-hydroxy-5-(benzofuranyl)pent-1-enyl)-5-oxocyclopentylsulfanyl]-propylsulfanyl}essigsäureisopropylester,
{3-[(1R,2S,3R)-2-((E)-4-Benzo[b]thiophen-3-yl-3-hydroxybut-1-enyl)-3-hydroxy-5-oxocyclopentylsulfanyl]propylsulfanyl}essigsäuremethylester,
{3-[(1R,2S,3R)-2-((E)-4-Benzo[b]thiophen-3-yl-3-hydroxybut-1-enyl)-3-hydroxy-5-oxocyclopentylsulfanyl]propylsulfanyl}essigsäure und
{3-[(1R,2S,3R)-3-Hydroxy-2-((S)-(E)-3-(hydroxy)-3-(methyl)-5-(benzothienyl)pent-1-enyl)-5-oxocyclopentylsulfanyl]propylsulfanyl}essigsäuremethylester.

## Revendications

1. Utilisation d'un composé représenté par la formule générale 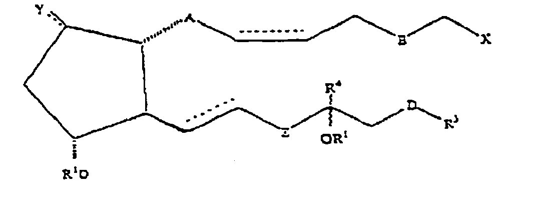 dans laquelle les lignes hachurées représentent la configuration α, un triangle représente la configuration β, une ligne ondulée représente soit la configuration α, soit la configuration β, et une ligne pointillée représente la présence ou l'absence d'une double liaison;
A et B sont choisis indépendamment dans le groupe constitué par O, S et CH₂, à condition qu'au moins l'un des A et B soit S;
D représente une liaison covalente ou CH₂, O, S ou NH;
X est CO₂R, CONR₂, CH₂OR, P(O)(OR)₂, CONRSO₂R, SONR₂ ou 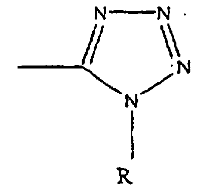 Y est O, OH, OCOR², halogène ou cyano;
Z est CH₂ ou une liaison covalente;
R est H ou R²_{;}
R¹ est H, R², phényle ou COR²,
R² est alcényle ou alkyle inférieur en C₁-C₅,
R³ est benzothiényle, benzofuranyle ou leurs dérivés substitués, les substituants pouvant être choisis dans le groupe constitué par alkyle en C₁-C₅, halogène, CF₃, CN, NO₂, NR₂, CO₂R et OR et
R⁴ est hydrogène ou alkyle inférieur en C₁-C₅,
pour la fabrication d'un médicament destiné au traitement de l'hypertension oculaire ou du glaucome.

2. Utilisation selon la revendication 1, dans laquelle ledit composé est représenté par la formule générale II:

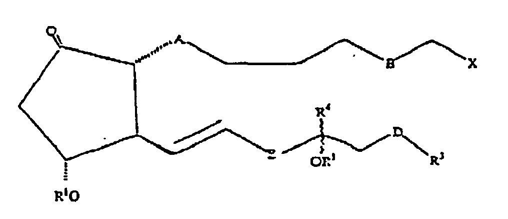

3. Utilisation selon la revendication 2, dans laquelle ledit composé est représenté par la formule générale III:

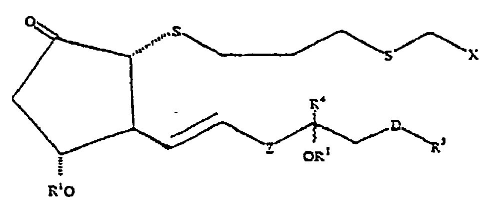

4. Utilisation selon la revendication 1, dans laquelle Z représente une liaison covalente.

5. Utilisation selon la revendication 1, dans laquelle D représente une liaison covalente ou est CH₂.

6. Utilisation selon la revendication 1, dans laquelle X est CO₂R.

7. Utilisation selon la revendication 6, dans laquelle R est choisi dans le groupe constitué par H, méthyle, isopropyle et n-propényle.

8. Utilisation selon la revendication 1, dans laquelle R est H ou n-propényle.

9. Utilisation selon la revendication 1, dans laquelle R¹ est H.

10. Utilisation selon la revendication 1, dans laquelle D est CH₂.

11. Utilisation selon la revendication 10, dans laquelle R³ est benzo[b]thiényle ou 3-chlorobenzo[b]thiényle.

12. Utilisation selon la revendication 1, dans laquelle ledit composé est choisi dans le groupe constitué par:
le {3-[(1R,2S,3R)-3-hydroxy-2-((S)-(E)-3-hydroxy-5-(benzothiényl)pent-1-ényl)-5-oxocyclopentylsulfanyl]propylsulfanyl}acétate de méthyle;
l'acide {3-[(1R,2S,3R)-3-hydroxy-2-((S)-(E)-3-hydroxy-5-(benzothiényl)pent-1-ényl)-5-oxocyclopentylsulfanyl]propylsulfanyl}acétique;
le {3-[(1R,2S,3R)-3-hydroxy-2-((S)-(E)-3-hydroxy-5-(benzothiényl)pent-1-ényl)-5-oxocyclopentylsulfanyl]propylsulfanyl}acétate d'isopropyle;
le {3-[(1R,2S,3R)-3-hydroxy-2-((S)-(E)-3-hydroxy-5-(benzofuranyl)pent-1-ényl)-5-oxocyclopentylsulfanyl]propylsulfanyl}acétate de méthyle;
l'acide {3-[(1R,2S,3R)-3-hydroxy-2-((S)-(E)-3-hydroxy-5-(benzofuranyl)pent-1-ényl)-5-oxocyclopentylsulfanyl]propylsulfanyl}acétique;
le {3-[(1R,2S,3R)-3-hydroxy-2-((S)-(E)-3-hydroxy-5-(benzofuranyl)pent-1-ényl)-5-oxocyclopentylsulfanyl]propylsulfanyl}acétate d'isopropyle;
le {3-[(1R,2S,3R)-2-((E)-4- benzo[b]thiophén-3-yl-3-hydroxybut-1-ényl)-3-hydroxy-5-oxocyclopentylsulfanyl]propylsulfanyl}acétate de méthyle;
l'acide {3-[(1R,2S,3R)-2-((E)-4-benzo[b]thiophén-3-yl-3-hydroxybut-1-ényl)-3-hydroxy-5-oxocyclopentylsulfanyl]propylsulfanyl}acétique; et
le {3-[(1R,2S,3R)-3-hydroxy-2-((S)-(E)-3-(hydroxy)-3-(méthyl)-5-(benzothiényl)pent-1-ényl)-5-oxocyclopentylsulfanyl]propylsulfanyl}acétate de méthyle.

13. Utilisation selon la revendication 1, dans laquelle D est CH₂ et Z représente une liaison covalente.

14. Utilisation selon la revendication 1, dans laquelle R⁴ est un hydrogène ou un méthyle.

15. Utilisation selon la revendication 1, dans laquelle R⁴ est un hydrogène.

16. Solution ophtalmique comprenant une quantité thérapeutiquement efficace d'un composé représenté par la formule générale I 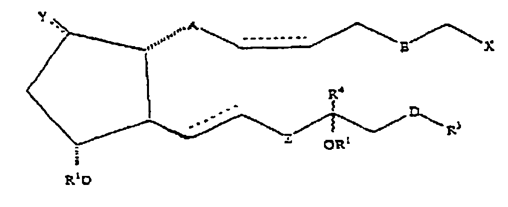 dans laquelle les lignes hachurées représentent la configuration α, un triangle représente la configuration β, une ligne ondulée représente soit la configuration α, soit la configuration β, et une ligne pointillée représente la présence ou l'absence d'une double liaison;
A et B sont choisis indépendamment dans le groupe constitué par O, S et CH₂, à condition qu'au moins l'un des A et B soit S;
D représente une liaison covalente ou CH₂, O, S ou NH;
X est CO₂R, CONR₂, CH₂OR, P(O)(OR)₂, CONRSO₂R, SONR₂ ou 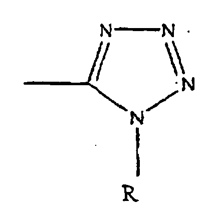 Y est O, OH, OCOR², un halogène ou un cyano;
Z est CH₂ ou une liaison covalente;
R est H ou R²;
R¹ est H, R², phényle ou COR²,
R² est alcényle ou alkyle inférieur en C₁-C₅,
R³ est benzothiényle, benzofuranyle ou leurs dérivés substitués, les substituants pouvant être choisis dans le groupe constitué par alkyle en C₁-C_{5,} halogène, CF₃, CN, NO₂, NR₂, CO₂R et OR et
R⁴ est hydrogène ou alkyle en C₁-C₅,
en mélange avec un véhicule liquide non toxique, acceptable sur le plan ophtalmologique, conditionné dans un récipient approprié pour une application dosée, le pH de la solution ophtalmique étant maintenu entre 6,5 et 7,2 à l'aide d'une solution tampon appropriée.

17. Produit pharmaceutique, comprenant un récipient adapté pour distribuer le contenu dudit récipient sous une forme dosée; et une solution ophtalmique selon la revendication 16 dans ledit récipient.

18. Nouveau composé choisi dans le groupe constitué par:
le {3-[(1R,2S,3R)-3-hydroxy-2-((S)-(E)-3-hydroxy-5-(benzothiényl)pent-1-ényl)-5-oxocyclopentylsulfanyl]propylsulfanyl}acétate de méthyle;
l'acide {3-[(1R,2S,3R)-3-hydroxy-2-((S)-(E)-3-hydroxy-5-(benzothiényl)pent-1-ényl)-5-oxocyclopentylsulfanyl]propylsulfanyl}acétique;
le {3-[(1R,2S,3R)-3-hydroxy-2-((S)-(E)-3-hydroxy-5-(benzothiényl)pent-1-ényl)-5-oxocyclopentylsulfanyl]propylsulfanyl}acétate d'isopropyle;
le {3-[(1R,2S,3R)-3-hydroxy-2-((S)-(E)-3-hydroxy-5-(benzofuranyl)pent-1-ényl)-5-oxocyclopentylsulfanyl]propylsulfanyl}acétate de méthyle;
l'acide {3-[(1R,2S,3R)-3-hydroxy-2-((S)-(E)-3-hydroxy-5-(benzofuranyl)pent-1-ényl)-5-oxocyclopentylsulfanyl]propylsulfanyl}acétique;
le {3-[(1R,2S,3R)-3-hydroxy-2-((S)-(E)-3-hydroxy-5-(benzofuranyl)pent-1-ényl)-5-oxocyclopentylsulfanyl]propylsulfanyl}acétate d'isopropyle;
le {3-[(1R,2S,3R)-2-((E)-4-benzo[b]thiophén-3-yl-3-hydroxybut-1-ényl)-3-hydroxy-5-oxocyclopentylsulfanyl]propylsulfanyl}acétate de méthyle;
l'acide {3-[(1R,2S,3R)-2-((E)-4- benzo[b]thiophén-3-yl-3-hydroxybut-1-ényl)-3-hydroxy-5-oxocyclopentylsulfanyl]propylsulfanyl}acétique; et
le {3-[(1R,2S,3R)-3-hydroxy-2-((S)-(E)-3-(hydroxy)-3-(méthyl)-5-(benzothiényl)pent-1-ényl)-5-oxocyclopentylsulfanyl]propylsulfanyl}acétate de méthyle.
